(19) Europäisches Patentamt
European Patent Office
Office européen des brevets

(11) Publication number: 0 046 468
A1

# (12) EUROPEAN PATENT APPLICATION

(21) Application number: 80302909.9

(22) Date of filing: 21.08.80

(51) Int. Cl.³: C 07 D 241/44
C 07 D 413/12, A 01 N 43/60
A 01 N 43/76

(43) Date of publication of application:
03.03.82 Bulletin 82/9

(84) Designated Contracting States:
CH DE FR GB IT LI

(71) Applicant: NISSAN CHEMICAL INDUSTRIES LTD.
7-1, Kandanishikicho-3-chome Chiyoda-ku
Tokyo(JP)

(72) Inventor: Ura, Yasukazu Nissan Chemical Industries Ltd.
Chuo Kenkyusho, 722-1 Tsuboicho
Funabashi-shi Chiba-ken(JP)

(72) Inventor: Sakata, Gozyo Nissan Chemical Industries Ltd.
Chuo Kenkyusho, 722-1 Tsuboicho
Funabashi-shi Chiba-ken(JP)

(72) Inventor: Makino, Kenzi Nissan Chemical Industries Ltd.
Chuo Kenkyusho, 722-1 Tsuboicho
Funabashi-shi Chiba-ken(JP)

(72) Inventor: Kawamura, Yasuo Nissan Chemical Industries Ltd.
Chuo Kenkyusho, 722-1 Tsuboicho
Funabashi-shi Chiba-ken(JP)

(72) Inventor: Kawamura, Yuzi Nissan Chemical Industries Ltd.
Seibutsu Kagaku Kenkyusho No. 1470, Oaza-Shiraoka
Shiraoka-machi Minami Saitama-gunSaitama(JP)

(72) Inventor: Ikai, Takasi Nissan Chemical Industries Ltd.
Seibutsu Kagaku Kenkyusho No. 1470, Oaza-Shiraoka
Shiraoka-machi Minami Saitama-gunSaitama(JP)

(72) Inventor: Oguti, Tosihiko Nissan Chemical Industries Ltd.
Seibutsu Kagaku Kenkyushi No. 1470, Oaza-Shiraoka
Shiraoka-machi Minami Saitama-gunSaitama(JP)

(74) Representative: Warden, John C. et al,
R.G.C. Jenkins & Co. Chancery House 53/64 Chancery Lane
London WC2A 1QU(GB)

## (54) Quinoxaline derivatives and herbicidal composition.

(57) Novel quinoxaline derivatives are disclosed having the general formula:

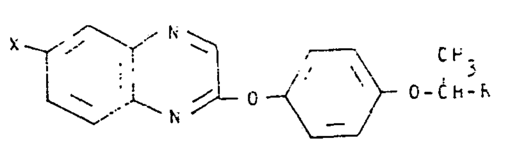

(I)

wherein X represents a $-CF_3$ or $-CH_3$ group and R represents one of a wide variety of possible substituents.

These compounds have selective herbicidal activity against gramineous weeds, without phytotoxicity towards broad-leaved plants. Typical herbicidal compositions containing these compounds are also described.

EP 0 046 468 A1

QUINOXALINE DERIVATIVES AND HERBICIDAL COMPOSITION

The present invention relates to novel quinoxaline derivatives and herbicidal compositions containing the same.

Although a wide range of herbicidal compounds have been used in the past to reduce labour works and to improve the productivities of agricultural and horticultural crop plants, there is still a need for novel herbicides having superior herbicidal characteristics. Herbicides for agricultural and horticultural purposes are preferably compounds small doses of which selectively control specific weeds without having any toxic effect on the crop plants. The known herbicides do not always have these optimum herbicidal characteristics.

The inventors have worked on the development of useful new herbicides and in particular have studied the herbicidal characteristics of various heterocyclic compounds.

Substituted pyridyloxyphenoxy fatty acid herbicides are examples of heterocyclic ether-type phenoxy fatty acid derivatives having herbicidal activity, and are disclosed in Japan Unexamined Patent Publication No. 106735/1976.

BAD ORIGINAL

Benzimidazole, benzthiazole, and benzoxazole derivatives and their herbicidal effects have been described in Japanese Unexamined Patent Publication No. 40767/1978.

The present invention provides novel quinoxaline derivatives having the formula:

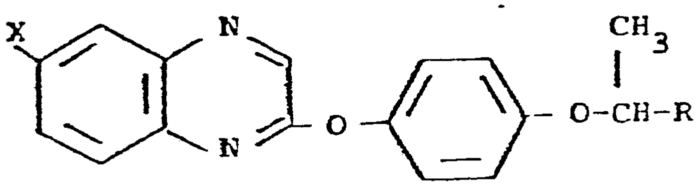

(I)

wherein X represents a $-CF_3$ or $-CH_3$ group and R represents a group of formula 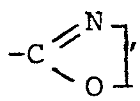, 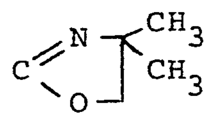, $-CH=CHCOOR^2$, ($R^2$ represents a $C_1 - C_4$ alkyl group) -CN, $-CH_2OH$ or -COR' wherein $R^1$ represents a group of formula -OH, -O- alkali metal, -O-lower alkyl group, -O-halogenoalkyl group, -O-cyanoalkyl group, -O-lower alkoxyalkyl group, -O-lower alkenyl group, -O-lower alkynyl group, -O-cyclohexyl group, -O-phenyl group, 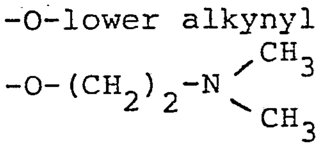, 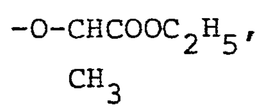, $-O-(CH_2)_2SCH_3$,

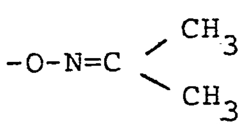, $-S-R^3$ ($R^3$ represents a lower alkyl or phenyl group), or 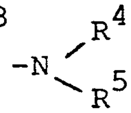, ($R^4$ and $R^5$ respectively represent a hydrogen atom, a lower alkyl group, a phenyl group or $-CH_2COOC_2H_5$).

BAD ORIGINAL

The quinoxaline derivatives having the formula (I) defined above are novel compounds having characteristic herbicidal properties, and are particularly effective for controlling gramineous weeds having no phytotoxicity towards broad-leaf crop plants or broad-leaf weeds, especially in a post-emergence treatment. Such unique characteristics have not previously been found.

Typical compounds of the present invention having the formula (I) are shown in Table (1) together with their physical properties. The scope of the present invention is not of course limited to the particular compounds shown in Table 1.

BAD ORIGINAL

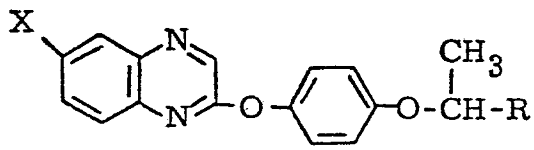

| Comp. No. | X | R | Physical Property |
|---|---|---|---|
| 1 | $CF_3$ | $-COOH$ | mp 141-143°C W.C. |
| 2 | " | $-COONa$ | mp >250°C W.C. |
| 3 | " | $-COOCH_3$ | mp 93-95°C W.C. |
| 4 | " | $-COOC_2H_5$ | mp 68-70°C W.C. |
| 5 | " | $-COOC_3H_7$-i | mp 121-123°C W.C. |
| 6 | " | $-COOC_4H_9$-sec | W.C. |
| 7 | " | $-COO(CH_2)_2Cl$ | W.C. |
| 8 | " | $-COOCH_2CN$ | mp 115-117°C W.C. |
| 9 | " | $-COO(CH_2)_2OCH_3$ | mp 91-92°C W.C. |
| 10 | " | $-COO(CH_2)_2N(CH_3)_2$ | W.C. |
| 11 | " | $CH_3$<br>$-COOCHCOOC_2H_5$ | W.C. |
| 12 | " | $-COOCH_2CH{=}CH_2$ | mp 72-74°C W.C. |
| 13 | " | $-COOCH_2C{\equiv}CH$ | mp 92-93°C W.C. |
| 14 | " | -COO-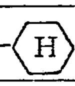 | mp 78-79°C W.C. |
| 15 | " | -COO-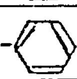 | mp 107-109°C W.C. |
| 16 | " | $-COSCH_3$ | mp 109-110°C W.C. |
| 17 | " | $-CON(CH_3)_2$ | mp 121-123°C W.C. |
| 18 | " | -CONH-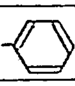 | W.C. |
| 19 | " | $-CONHCH_2COOC_2H_5$ | W.C. |

| Comp.No. | X | R | Physical Property | |
|---|---|---|---|---|
| 20 | $CF_3$ | 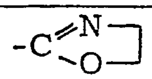 | mp 153-155°C | W.C. |
| 21 | " | 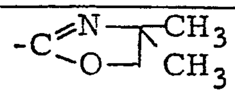 | | W.C. |
| 22 | " | $-CH=CHCOOCH_3$ | $N_D^{20}$=1.5485 | Colorless liq. |
| 23 | " | $-CH=CHCOOC_2H_5$ | $N_D^{20}$=1.5433 | Colorless liq. |
| 24 | " | -CN | mp 108-109°C | W.C. |
| 25 | " | $-CH_2OH$ | | W.C. |
| 26 | $CH_3$ | -COOH | | W.C. |
| 27 | " | $-COOCH_3$ | mp 118-120°C | W.C. |
| 28 | " | $-COOC_2H_5$ | mp 91-92°C | W.C. |
| 29 | " | $-COOC_3H_7$-i | mp 102-103°C | W.C. |
| 30 | " | $-COO(CH_2)_2SCH_3$ | | W.C. |
| 31 | " | 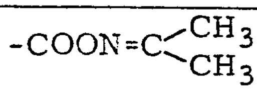 | | W.C. |
| 32 | " | -COS-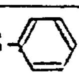 | | W.C. |

The compound (I) of the present invention can be produced by the following processes.

A) The compound of the present invention can be produced by a condensation of a compound having the formula

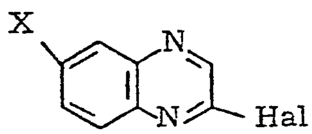

(II)

wherein X is defined above; Hal designates a halogen atom; with 4-hydroxyphenoxy fatty acid derivative having the formula

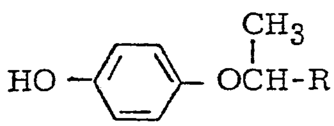

(III)

wherein R is defined above, in the presence of an inorganic or organic base such as sodium hydroxide, potassium hydroxide or potassium carbonate, at suitable temperature.

The reaction can be carried out in an inert solvent such as dimethylformamide, dimethylsulfoxide, or acetonitrile.

B) The compound of the present invention can be produced by a condensation of a compound having the formula (II) with a hydroquinone monobenzyl ether having the formula

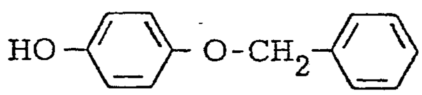

(IV)

in the presence of an inorganic or organic base to produce a compound having the formula

BAD ORIGINAL

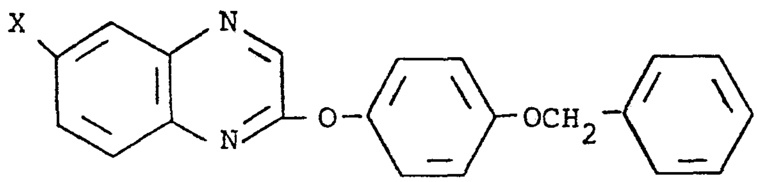

(V)

wherein X is as defined above, followed by a hydrogenation of the product with a catalyst such as palladium-carbon to bring about a debenzylation and obtain a compound having the formula

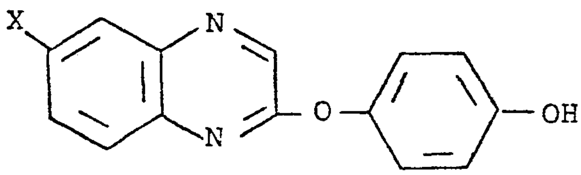

(VI)

and then a condensation of the product with a haloalkyl derivative having the formula

Hal-CH-R
|
$CH_3$

(VII)

in the presence of an inorganic or organic base such potassium carbonate in a polar organic solvent such as methyl ethyl ketone, acetonitrile or dimethyl-formamide.

C) The product obtained by the process A) or B) can be converted into other compounds of the present invention by hydrolysis, esterification, ester interchange, salt or amidation.

In the esterification, it is possible to use a conventional coupling agent but there are also coupling

BAD ORIGINAL

agents particularly suitable for this specific reaction such as imido coupling agents especially dicyclohexyl carboimide. The concentrations of the reagents, the temperatures in the reactions and kind of inert solvent can be selected as desired.

In the process A), the reaction is preferably carried out at 50 to 200°C, especially at 80 to 100°C, at a molar ratio of the compound (II) to the 4-hydroxyphenoxy derivative (III) of 1:0.2 to 5.0, preferably 1:0.5 to 2.0 and especially 1:0.8 to 1.5. The inorganic or organic base can be any base which is suitable for the condensation of the compound (II) and the compound (III). The concentration of the starting materials in the inert solvent can suitably be in a range of 5 to 50 wt.%, preferably 10 to 30 wt.%.

In the process B), the reaction is preferably carried out at 50 to 200°C, especially at 100 to 150°C at a molar ratio of compound (II) to the hydroquinone monobenzyl ether (IV) of 1:0.2 to 5.0, preferably 1:0.5 to 2.0 and especially 1:0.8 to 1.5. The inorganic or organic base can be any base which is suitable for the condensation of the compound (II) and the compound (IV). The reaction is preferably carried out in an inert solvent at a concentration of the starting material of 5 to 50 wt.%, preferably 10 to 30 wt.%.

BAD ORIGINAL

The hydrogenation of the resulting intermediate (V) is carried out in conditions suitable for the debenzylation to give the compound (VI). The hydrogen pressure is preferably in the range of 1 to 5 atm., more preferably 1 to 2 atm.

The reaction of the compound (VI) with the compound (VII) is preferably carried out at 80 to 100°C at a molar ratio of the compound (VI) to the compound (VII) of 1:0.2 to 5.0, preferably 1:0.5 to 2.0 and especially 1:0.8 to 1.5. The inorganic or organic base can be the same as that used in the previous reaction. The concentration of the starting materials in the inert solvent can be in a range of 5 to 50 wt.% preferably 10 to 30 wt.%.

BAD ORIGINAL

In the process C), the conditions of the hydrolysis, the esterification, the ester interchange, the neutralization and the amidation can be selected as desired. These conditions can be considered by a person skilled in the art.

Certain examples for the preparations of the present invention will be described.

Preparation 1:

Methyl 2-[4-(6-trifluoromethyl-2-quinoxalyloxy)phenoxy] propionate:
(Compound 3)

In 100 ml. of acetonitrile, 2.3 g. (0.01 mol) of 2-chloro-6-trifluoromethylquinoxaline, 2.2 g.(0.011 mol) of methyl 2-(4'-hydroxyphenoxy)-propionate and 2.0 g.(0.014 mol) of potassium carbonate were dissolved and the mixture was refluxed for 6 hours. After the reaction, the precipitated salt was separated by a filtration and the filtrate was concentrated and dried. The residue was dissolved in chloroform, and the chloroform layer was washed with 5 % aqueous solution of sodium hydroxide and with water. The chloroform layer was concentrated and dried. The residue was washed with n-hexane to obtain 3.4 g. of white crystal of the object compound having a melting compound 93 to 95°C.

Preparation 2:

2-[1-{4-(6-trifluoromethyl-2-quinoxalyloxy)-phenoxy}-ethyl]-2-oxazoline:
(Compound 20)

In 20 ml. of dimethyl-formamide, 1.5 g.(0.0049 mol) of 6-trifluoromethyl-2-(4-hydroxyphenoxy)-quinoxaline, 0.72 g.(0.0054 mol) of 2-(1-chloroethyl)-2-oxazoline and 0.81 g.(0.0059 mol) of potassium carbonate were dissolved and the mixture was heated at 90°C for 8 hours with stirring. After the reaction, the reaction mixture was charged into 300 ml. of water and the product was extracted with 600 ml. of benzene. The benzene layer was washed

BAD ORIGINAL

with 5 % aqueous solution of sodium hydroxide and with water and dehydrated over anhydrous sodium sulfate. Benzene was distilled off under a reduced pressure to obtain a crude product. The crude product was washed with n-hexane to obtain 0.49 g. of white solid of the object compound having a melting point of 153 to 155°C(yield of 20 %).

Preparation 3:

Methyl γ-methyl-γ-[4-(6-trifluoromethyl-2-quinoxalyloxy) phenoxy]crotonate:
(Compound 22)

In 150 mℓ. of methyl ethyl ketone, 3.1 g.(0.01 mol) of 6-trifluoromethyl-2-(4'-hydroxyphenoxy)quinoxaline, 2.0 g.(0.01 mol) of methyl γ-bromo-γ-methylcrotonate and 2.0 g.(0.014 mol) of potassium carbonate were dissolved and the mixture was refluxed for 8 hours. After the reaction, the precipitated salt was filtered and the filtrate was concentrated and dried.

The residue was dissolved in chloroform. The chloroform layer was washed with a small amount of 5 % aqueous solution of sodium hydroxide and then, washed with water and dehydrated over anhydrous sodium sulfate and concentrated and dried. The residue was purified by a column chromatography with silica gel and chloroform to obtain 3.6 g. of a pale yellow liquid of the object compound having a refractive index $N_D^{20}$=1.5485 (yield of 85 %)

BAD ORIGINAL

Preparation 4:

Methyl 2-[4-(6-methyl-2-quinoxalyloxy)phenoxy] propionate:
(Compound 29)

In 50 mℓ. of acetonitrile, 1.79 g.(0.01 mol) of 2-chloro-6-methyl quinoxaline, 2.69 g.(0.012 mol) of isopropyl 2-(4'-hydroxyphenoxy)propionate and 2.0 g.(0.014 mol) of potassium carbonate were dissolved and the mixture was refluxed for about 10 hours. After the reaction, the precipitated salt was separated by a filtration and the filtrate was distilled under a reduced pressure. The residual oily product was dissolved in chloroform and chloroform layer was washed with 5 % aqueous solution of sodium hydroxide and with water, and dehydrated over anhydrous sodium sulfate. Chloroform was distilled off under a reduced pressure and the crude crystal was washed with a mixed solvent of ether and n-hexane to obtain 2.6 g.(71 %) of white crystal of the object compound having a melting point of 102.0 to 103.0°C.(yield of 71 %)

The compound of the present invention can be used as a herbicidal composition.

In the preparation of the herbicidal compositions, the compound of the present invention can be uniformly mixed with or dissolved in suitable adjuvants such as solid carrier such as clay, talc, bentonite, diatomaceous earth; liquid carrier such as water,

BAD ORIGINAL

alcohols (methanol, ethanol etc.), aromatic hydrocarbons (benzene, toluene, xylene etc.) chlorinated hydrocarbons, ethers, ketones, esters (ethyl acetate etc.), acid amides (dimethylformamide etc.) if desired, with an emulsifier, a dispersing agent, a suspending agent, a wetting agent, a spreader, or a stabilizer to form a solution, an emulsifiable concentrate, a wettable powder, a dust, a granule or a flowable suspension which is applied if desired, by diluting it with suitable diluent.

It is possible to combine the compound of the present invention with the other herbicide, or an insecticide, a fungicide, a plant growth regulator, a synergism agent.

Certain examples of the herbicidal compositions of the present invention will be illustrated. In the examples, the part means part by weight.

Solution:

| | |
|---|---|
| Active ingredient: | 5 to 75 wt.% preferably 10 to 50 wt.% especially 15 to 40 wt.% |
| Solvent: | 95 to 25 wt.% preferably 88 to 30 wt.% especially 82 to 40 wt.% |
| Surfactant: | 1 to 30 wt.% preferably 2 to 20 wt.% |

Emulsifiable concentrate:

| | |
|---|---|
| Active ingredient: | 2.5 to 50 wt.% preferably 5 to 45 wt.% especially 10 to 40 wt.% |
| Surfactant: | 1 to 30 wt.% preferably 2 to 25 wt.% especially 3 to 20 wt.% |
| Liquid carrier: | 20 to 95 wt.% preferably 30 to 93 wt.% especially 57 to 85 wt.% |

BAD ORIGINAL

Dust:

| | |
|---|---|
| Active ingredient: | 0.5 to 10 wt.% |
| Solid carrier: | 99.5 to 90 wt.% |

Flowable suspension:

| | |
|---|---|
| Active ingredient: | 5 to 75 wt.% preferably 10 to 50 wt.% |
| Water: | 94 to 25 wt.% preferably 90 to 30 wt.% |
| Surfactant: | 1 to 30 wt.% preferably 2 to 20 wt.% |

Wettable powder:

| | |
|---|---|
| Active ingredient: | 2.5 to 90 wt.% preferably 10 to 80 wt.% especially 20 to 75 wt.% |
| Surfactant: | 0.5 to 20 wt.% preferably 1 to 15 wt.% especially 2 to 10 wt.% |
| Solid carrier: | 5 to 90 wt.% preferably 7.5 to 88 wt.% especially 16 to 56 wt.% |

Granule:

| | |
|---|---|
| Active ingredient: | 0.5 to 30 wt.% |
| Solid carrier: | 99.5 to 70 wt.% |

The emulsifiable concentrate is prepared by dissolving the active ingredient in the liquid carrier with the surfactant. The wettable powder is prepared by admixing the active ingredient with the solid carrier and the surfactant and the mixture is pulverized.

The flowable suspension is prepared by suspending to disperse a pulverized active ingredient into an aqueous solution of a surfactant. The dust, the solution, the granule etc. are prepared by mixing the active ingredient with the adjuvant.

In the following compositions, the following adjuvants are used.

BAD ORIGINAL

| | |
|---|---|
| Sorpol-2680 | |
| POE-hormylnonylphenolether | 50 wt. parts |
| POE-nonylphenolether | 20 " |
| POE-sorbitan alkyl ester | 10 " |
| Ca-alkylbenzenesulfonate | 20 " |
| Sorpol-5039 | |
| POE-alkylarylether sulfate | 50 " |
| Silica hydrate | 50 " |
| Carplex | |
| Silica hydrate | 100 " |
| Zeeklite | |
| Clay | 100 " |
| Sorpol W-150 | |
| POE-nonylphenolether | 100 wt. parts |

Composition 1: Wettable powder:

| | |
|---|---|
| Active ingredient | 50 wt.parts |
| Zeeklite A | 46 " |
| Sorpol 5039 (Toho Chem.) | 2 " |
| Carplex | 2 " |

These components were uniformly mixed and pulverized to prepare a wettable powder. The wettable powder was diluted with water at 50 to 1,000 times and the diluted solution was sprayed at a dose of 5 to 1,000 g. of the active ingredient per 10 ares.

Composition 2: Emulsifiable concentrate:

| | |
|---|---|
| Active ingredient | 20 wt.parts |
| Xylene | 75 " |
| Sorpol 2680 (Toho Chem.) | 5 " |

BAD ORIGINAL

The components were uniformly mixed to prepare an emulsifiable concentrate. The emulsifiable concentrate was diluted with water at 50 to 1,000 times and the diluted solution was sprayed at a dose of 5 to 1000 g. of the active ingredient per 10 ares.

Composition 3: Aqueous solution:

| | |
|---|---|
| Active ingredient | 30 wt.parts |
| Sorpol W-150 (Toho Chem.) | 10 " |
| Water | 60 " |

The components were mixed to dissolve them and to prepare an aqueous solution. The aqueous solution was diluted with water at 50 to 1,000 times and the diluted solution was sprayed at a dose of 5 to 1000 g. of the active ingredient per 10 ares.

Composition 4: Wettable powder:

| | |
|---|---|
| Active ingredient | 30 wt.parts |
| Other herbicide | 20 " |
| Zeeklite A | 46 " |
| Sorpol 5039 (Toho Chem.) | 2 " |
| Carplex | 2 " |

BAD ORIGINAL

As the other herbicide, the following known herbicides were respectively used 2-(2, 4-dichlorophenoxy)propionic acid, 2, 4-dichlorophenoxyacetic acid, 3-(3-trifluoromethylphenyl)-1, 1-dimethylurea, 3-(4-methylphenethyloxyphenyl)-1-methyl-1-methoxy urea, 3-(methoxycarbonylamino)-phenyl-N-(3-methylphenyl) carbamate, 3-(ethoxycarbonylamino)-phenyl-N-phenylcarbamate, 3-isopropyl-1H-2, 1, 3-benzo thiadiazine-(4)-3H- one-2, 2-dioxide, 5-amino-4-chloro-2-phenylpyridazine-3-one, 3-cyclohexyl-5, 6-trimethyleneuracil, 2-chloro-4-ethylamino-6-isopropylamino-1, 3, 5-triazine, 2-chloro-4, 6-di(ethylamino)-1, 3, 5-triazine, 2-methylthio-4, 6-bis(isopropylamino)-1, 3, 5-triazine, 4-amino-4, 5-dihydro-3-methyl-6-phenyl-1, 2, 4-triazine-5-one, 4-amino-6-t-butyl-4, 5-dihydro-3-methylthio-1, 2, 4-triazine-5-one, 2-chloro-4-trifluoromethylphenyl-3'-ethoxy-4'-nitrophenyl ether or sodium-5-[2-chloro-4-(trifluoromethyl) phenoxy]-2-nitro benzoate.

It is also possible to combine the compound of the present invention with the other herbicidal compounds which are described in "Weed Control Handbook" (Vol. I 6th edition 1977; Vol. II 8th edition 1978) issued by the British Crop Protection Council edited by J.D. Fryer MA & R.J. Makepeace BSc. Blackwell Scientific Publication.

BAD ORIGINAL

The quinoxaline derivatives of the present invention have excellent herbicidal effect against various weeds, especially gramineous weeds in a soil treatment or in a foliage treatment, without any phytotoxicity to broad-leaf crop plants such as cotton, soybean, radish, cabbage, eggplant, tomato, sugar beet, ground nut, peas, beans, line seed, sun flower, safflower, potato, tabacco, alfalfa and onion. Therefore, the quinoxaline derivatives of the present invention are suitable for selective control of gramineous weeds in the cultivation of a broad leaf crop plant in both the agricultural and horticultural fields, especially up-land cultivation.

The quinoxaline derivatives of the present invention are also effective as herbicides for controlling various weeds in a wide variety of agricultural and horticultural fields such as up-land, paddy fields and orchards as well as non-cultivated lands such as playgrounds, vacant lands, and railway sidings and embankments.

The herbicidal composition usually contains 0.5 to 95 wt.% of the compound of the present invention as the active ingredient and the remainder of the adjuvants in the concentrated form. The exact dose of the compound of the present invention may depend upon weather conditions, soil conditions, the form of the composition, the season of application and the kind of a crop plant and kind of weed

BAD ORIGINAL

and is usually in a range of 1 to 5000 g. preferably 5 to 1000 g. of the active ingredient per 10 ares.

The herbicidal activities of the quinoxaline derivatives of the present invention will be illustrated in the following tests.

In the following tests, the herbicidal effects of the compounds of the present invention to gramineous weeds including rice are shown together with non-phytotoxicity of the same compounds to broad leaf crop plants as well as broad leaf weeds especially, non-phytotoxicity of the same compounds to broad leaf weeds in post-emergence. These remarkable selectivities have not been found by the other compounds.

Test 1: Tests for herbicidal effect in soil treatment:

Each plastic box having a length of 15 cm, a width of 22 cm and a depth of 6 cm was filled with a sterilized diluvium soil and seeds of rice (Oryza sativa), barnyard grass (Echinochloa crus-galli), large crab-grass (Digitaria adscendens), lambsquarters (Chenopodium ficifolium), common purslane (Postuloca oleracea), hairy galinsoga (Galinsoga ciliata), yellow cress (Rorippa atrovirens) were sown in a depth of about 1.5 cm. Each solution of each herbicidal composition was uniformly sprayed on the surface of the soil to give the specific dose of the active ingredient.

BAD ORIGINAL

0046468

The solution was prepared by diluting, with water, a wettable powder, an emulsifiable concentrate or a solution described in examples of the composition except varying the active ingredient. The solution was sprayed by a small spray. Three weeks after the treatment, the herbicidal effects to rice and various weeds were observed and rated by the following standard. The results are shown in Table 2.

Standard rating:

5: Growth control of more than 90% (substantial suppression)

4: Growth control of 70 to 90%

3: Growth control of 40 to 70%

2: Growth control of 20 to 40%

1: Growth control of 5 to 20%

0: Growth control of less than 5% (non-herbicidal effect)

Note:
Ri: Rice
Ba.: Barnyard grass
L.C.: Large crab grass
La. : Lambsquarters
C.P.: Common purslane
H.G.: Hairy galinsoga
Y.C.: Yellow cress

BAD ORIGINAL

Table 2

| Comp. No. | Dose of Comp. (g/a) | Ri | Ba | L.C. | La. | C.P. | H.G. | Y.C. |
|---|---|---|---|---|---|---|---|---|
| 1 | 100<br>50<br>25 | 5<br>5<br>5 | 5<br>5<br>5 | 5<br>5<br>5 | 1<br>0<br>0 | 1<br>0<br>0 | 1<br>0<br>0 | 1<br>0<br>0 |
| 2 | 100<br>50<br>25 | 5<br>5<br>5 | 5<br>5<br>5 | 5<br>5<br>5 | 2<br>1<br>0 | 2<br>1<br>0 | 1<br>0<br>0 | 1<br>0<br>0 |
| 3 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |
| 4 | 100<br>50<br>25 | 5<br>5<br>5 | 5<br>5<br>5 | 5<br>5<br>5 | 2<br>1<br>0 | 2<br>1<br>0 | 2<br>1<br>0 | 3<br>1<br>0 |
| 5 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |
| 6 | 100<br>50<br>25 | 5<br>5<br>5 | 5<br>5<br>5 | 5<br>5<br>5 | 2<br>0<br>0 | 2<br>1<br>0 | 2<br>0<br>0 | 2<br>1<br>0 |
| 7 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |
| 8 | 100<br>50<br>25 | 5<br>5<br>5 | 5<br>5<br>5 | 5<br>5<br>5 | 2<br>1<br>0 | 2<br>0<br>0 | 2<br>0<br>0 | 2<br>1<br>0 |
| 9 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |
| 10 | 100<br>50<br>25 | 5<br>5<br>5 | 5<br>5<br>5 | 5<br>5<br>5 | 2<br>0<br>0 | 2<br>1<br>0 | 2<br>1<br>0 | 2<br>1<br>0 |
| 11 | 100<br>50<br>25 | 5<br>5<br>5 | 5<br>5<br>5 | 5<br>5<br>5 | 2<br>1<br>0 | 2<br>1<br>0 | 2<br>0<br>0 | 2<br>0<br>0 |

Table 2 (continued)

| Comp. No. | Dose of Comp. (g/a) | Ri | Ba | L.C. | La. | C.P. | H.G. | Y.C. |
|---|---|---|---|---|---|---|---|---|
| 12 | 100<br>50<br>25 | 5<br>5<br>5 | 5<br>5<br>5 | 5<br>5<br>5 | 2<br>1<br>0 | 2<br>0<br>0 | 2<br>1<br>0 | 2<br>0<br>0 |
| 13 | 100<br>50<br>25 | 5<br>5<br>5 | 5<br>5<br>5 | 5<br>5<br>5 | 2<br>1<br>0 | 3<br>2<br>0 | 2<br>0<br>0 | 2<br>0<br>0 |
| 14 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |
| 15 | 100<br>50<br>25 | 5<br>5<br>5 | 5<br>5<br>5 | 5<br>5<br>5 | 2<br>1<br>0 | 1<br>0<br>0 | 1<br>0<br>0 | 2<br>1<br>0 |
| 16 | 100<br>50<br>25 | 5<br>5<br>5 | 5<br>5<br>5 | 5<br>5<br>5 | 2<br>1<br>0 | 2<br>1<br>0 | 2<br>1<br>0 | 3<br>2<br>0 |
| 17 | 100<br>50<br>25 | 5<br>5<br>5 | 5<br>5<br>5 | 5<br>5<br>5 | 3<br>1<br>0 | 3<br>1<br>0 | 2<br>1<br>0 | 2<br>1<br>0 |
| 18 | 100<br>50<br>25 | 5<br>5<br>5 | 5<br>5<br>5 | 5<br>5<br>5 | 1<br>0<br>0 | 2<br>1<br>0 | 2<br>1<br>0 | 1<br>0<br>0 |
| 19 | 100<br>50<br>25 | 5<br>5<br>5 | 5<br>5<br>5 | 5<br>5<br>5 | 1<br>0<br>0 | 1<br>0<br>0 | 1<br>0<br>0 | 1<br>0<br>0 |
| 20 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |
| 21 | 100<br>50<br>25 | 5<br>5<br>5 | 5<br>5<br>5 | 5<br>5<br>5 | 1<br>0<br>0 | 2<br>1<br>0 | 3<br>1<br>0 | 2<br>1<br>0 |
| 22 | 100<br>50<br>25 | 5<br>5<br>5 | 5<br>5<br>5 | 5<br>5<br>5 | 2<br>1<br>0 | 2<br>1<br>0 | 2<br>1<br>0 | 3<br>2<br>0 |

Table 2 (continued)

| Comp. No. | Dose of Comp. (g/a) | Ri | Ba | L.C. | La. | C.P. | H.G. | Y.C. |
|---|---|---|---|---|---|---|---|---|
| 23 | 100<br>50<br>25 | 5<br>5<br>5 | 5<br>5<br>5 | 5<br>5<br>5 | 2<br>1<br>0 | 2<br>0<br>0 | 2<br>1<br>0 | 2<br>1<br>0 |
| 24 | 100<br>50<br>25 | 5<br>5<br>5 | 5<br>5<br>5 | 5<br>5<br>5 | 2<br>1<br>0 | 2<br>1<br>0 | 2<br>1<br>0 | 1<br>0<br>0 |
| 25 | 100<br>50<br>25 | 5<br>5<br>5 | 5<br>5<br>5 | 5<br>5<br>5 | 3<br>1<br>0 | 2<br>1<br>0 | 2<br>1<br>0 | 2<br>1<br>0 |
| 26 | 100<br>50<br>25 | 5<br>5<br>5 | 5<br>5<br>5 | 5<br>5<br>5 | 1<br>0<br>0 | 1<br>0<br>0 | 2<br>1<br>0 | 1<br>0<br>0 |
| 27 | 100<br>50<br>25 | 5<br>5<br>5 | 5<br>5<br>5 | 5<br>5<br>5 | 1<br>0<br>0 | 2<br>1<br>0 | 2<br>1<br>0 | 1<br>0<br>0 |
| 28 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |
| 29 | 100<br>50<br>25 | 5<br>5<br>5 | 5<br>5<br>5 | 5<br>5<br>5 | 2<br>1<br>0 | 1<br>0<br>0 | 2<br>1<br>0 | 2<br>1<br>0 |
| 30 | 100<br>50<br>25 | 5<br>5<br>5 | 5<br>5<br>5 | 5<br>5<br>5 | 1<br>0<br>0 | 1<br>0<br>0 | 2<br>1<br>0 | 1<br>0<br>0 |
| 31 | 100<br>50<br>25 | 5<br>5<br>5 | 5<br>5<br>5 | 5<br>5<br>5 | 2<br>1<br>0 | 2<br>1<br>0 | 3<br>2<br>0 | 2<br>1<br>0 |
| 32 | 100<br>50<br>25 | 5<br>5<br>5 | 5<br>5<br>5 | 5<br>5<br>5 | 3<br>2<br>0 | 2<br>1<br>0 | 1<br>0<br>0 | 2<br>1<br>0 |

Test 2: Tests for herbicidal effect in foliage treatment:

Each plastic box having a length of 15 cm, a width of 22 cm, a depth of 6 cm was filled with a sterilized diluvium soil and seeds of rice, barnyard grass, large crab-grass, lambsquarters common purslane, hairy galinsoga, yellow cress and tomato were sown in a form of spots in a depth of about 1.5 cm. When the weeds were grown to 2 to 3 leaf stage, each solution of each herbicidal composition was uniformly sprayed to foliages at each dose of each active ingredient shown in Table 3. The solution was prepared by diluting, with water, a wettable powder, an emulsifiable concentrate or a solution described in examples of the composition except varying the active ingredient and the solution was uniformly sprayed by a small spray on all of foliages of the plants.

Two weeks after the spray treatment, the herbicidal effects to the weeds and tomato were observed and rated by the standard shown in Test 1. The results are shown in Table 3.

BAD ORIGINAL

Table 3

| Comp. No. | Dose of Comp. (g/a) | Ri | Ba | L.C. | La. | C.P. | H.G. | Y.C. |
|---|---|---|---|---|---|---|---|---|
| 1 | 100<br>50<br>25 | 5<br>5<br>5 | 5<br>5<br>5 | 5<br>5<br>5 | 2<br>0<br>0 | 2<br>0<br>0 | 2<br>0<br>0 | 1<br>0<br>0 |
| 2 | 100<br>50<br>25 | 5<br>5<br>5 | 5<br>5<br>5 | 5<br>5<br>5 | 1<br>0<br>0 | 1<br>0<br>0 | 1<br>0<br>0 | 1<br>0<br>0 |
| 3 | 100<br>50<br>25 | 5<br>5<br>5 | 5<br>5<br>5 | 5<br>5<br>5 | 1<br>0<br>0 | 1<br>0<br>0 | 1<br>0<br>0 | 1<br>0<br>0 |
| 4 | 100<br>50<br>25 | 5<br>5<br>5 | 5<br>5<br>5 | 5<br>5<br>5 | 1<br>0<br>0 | 1<br>0<br>0 | 1<br>0<br>0 | 1<br>0<br>0 |
| 5 | 100<br>50<br>25 | 5<br>5<br>5 | 5<br>5<br>5 | 5<br>5<br>5 | 2<br>1<br>0 | 2<br>1<br>0 | 2<br>1<br>0 | 1<br>0<br>0 |
| 6 | 100<br>50<br>25 | 5<br>5<br>5 | 5<br>5<br>5 | 5<br>5<br>5 | 2<br>1<br>0 | 3<br>2<br>0 | 2<br>1<br>0 | 2<br>1<br>0 |
| 7 | 100<br>50<br>25 | 5<br>5<br>5 | 5<br>5<br>5 | 5<br>5<br>5 | 3<br>1<br>0 | 2<br>1<br>0 | 2<br>0<br>0 | 2<br>1<br>0 |
| 8 | 100<br>50<br>25 | 5<br>5<br>5 | 5<br>5<br>5 | 5<br>5<br>5 | 2<br>0<br>0 | 1<br>0<br>0 | 1<br>0<br>0 | 2<br>1<br>0 |
| 9 | 100<br>50<br>25 | 5<br>5<br>5 | 5<br>5<br>5 | 5<br>5<br>5 | 1<br>0<br>0 | 2<br>1<br>0 | 1<br>0<br>0 | 2<br>1<br>0 |
| 10 | 100<br>50<br>25 | 5<br>5<br>5 | 5<br>5<br>5 | 5<br>5<br>5 | 1<br>0<br>0 | 1<br>0<br>0 | 1<br>0<br>0 | 1<br>0<br>0 |
| 11 | 100<br>50<br>25 | 5<br>5<br>5 | 5<br>5<br>5 | 5<br>5<br>5 | 1<br>0<br>0 | 2<br>1<br>0 | 1<br>0<br>0 | 2<br>1<br>0 |

Table 3 (continued)

| Comp. No. | Dose of Comp. (g/a) | Ri | Ba | L.C. | La. | C.P. | H.G. | Y.C. |
|---|---|---|---|---|---|---|---|---|
| 12 | 100<br>50<br>25 | 5<br>5<br>5 | 5<br>5<br>5 | 5<br>5<br>5 | 2<br>1<br>0 | 3<br>2<br>0 | 2<br>1<br>0 | 2<br>1<br>0 |
| 13 | 100<br>50<br>25 | 5<br>5<br>5 | 5<br>5<br>5 | 5<br>5<br>5 | 3<br>1<br>0 | 2<br>0<br>0 | 2<br>1<br>0 | 1<br>0<br>0 |
| 14 | 100<br>50<br>25 | 5<br>5<br>5 | 5<br>5<br>5 | 5<br>5<br>5 | 2<br>1<br>0 | 1<br>0<br>0 | 1<br>0<br>0 | 1<br>0<br>0 |
| 15 | 100<br>50<br>25 | 5<br>5<br>5 | 5<br>5<br>5 | 5<br>5<br>5 | 1<br>0<br>0 | 2<br>1<br>0 | 2<br>1<br>0 | 1<br>0<br>0 |
| 16 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |
| 17 | 100<br>50<br>25 | 5<br>5<br>5 | 5<br>5<br>5 | 5<br>5<br>5 | 2<br>1<br>0 | 3<br>1<br>0 | 2<br>0<br>0 | 2<br>1<br>0 |
| 18 | 100<br>50<br>25 | 5<br>5<br>5 | 5<br>5<br>5 | 5<br>5<br>5 | 1<br>0<br>0 | 2<br>1<br>0 | 2<br>1<br>0 | 2<br>1<br>0 |
| 19 | 100<br>50<br>25 | 5<br>5<br>5 | 5<br>5<br>5 | 5<br>5<br>5 | 1<br>0<br>0 | 2<br>0<br>0 | 2<br>0<br>0 | 3<br>2<br>0 |
| 20 | 100<br>50<br>25 | 5<br>5<br>5 | 5<br>5<br>5 | 5<br>5<br>5 | 1<br>1<br>0 | 1<br>0<br>0 | 1<br>0<br>0 | 1<br>1<br>0 |
| 21 | 100<br>50<br>25 | 5<br>5<br>5 | 5<br>5<br>5 | 5<br>5<br>5 | 1<br>0<br>0 | 2<br>1<br>0 | 2<br>1<br>0 | 1<br>0<br>0 |
| 22 | 100<br>50<br>25 | 5<br>5<br>5 | 5<br>5<br>5 | 5<br>5<br>5 | 2<br>1<br>0 | 2<br>1<br>0 | 2<br>1<br>0 | 2<br>1<br>0 |

Table 3 (continued)

| Comp. No. | Dose of Comp. (g/a) | Ri | Ba | L.C. | La. | C.P. | H.G. | Y.C. |
|---|---|---|---|---|---|---|---|---|
| 23 | 100<br>50<br>25 | 5<br>5<br>5 | 5<br>5<br>5 | 5<br>5<br>5 | 1<br>0<br>0 | 2<br>1<br>0 | 2<br>1<br>0 | 2<br>1<br>0 |
| 24 | 100<br>50<br>25 | 5<br>5<br>5 | 5<br>5<br>5 | 5<br>5<br>5 | 2<br>1<br>0 | 3<br>1<br>0 | 2<br>1<br>0 | 1<br>0<br>0 |
| 25 | 100<br>50<br>25 | 5<br>5<br>5 | 5<br>5<br>5 | 5<br>5<br>5 | 2<br>1<br>0 | 3<br>2<br>0 | 2<br>1<br>0 | 2<br>1<br>0 |
| 26 | 100<br>50<br>25 | 5<br>5<br>5 | 5<br>5<br>5 | 5<br>5<br>5 | 2<br>1<br>0 | 2<br>1<br>0 | 2<br>0<br>0 | 2<br>1<br>0 |
| 27 | 100<br>50<br>25 | 5<br>5<br>5 | 5<br>5<br>5 | 5<br>5<br>5 | 2<br>1<br>0 | 2<br>0<br>0 | 2<br>1<br>0 | 2<br>1<br>0 |
| 28 | 100<br>50<br>25 | 5<br>5<br>5 | 5<br>5<br>5 | 5<br>5<br>5 | 1<br>0<br>0 | 1<br>0<br>0 | 2<br>1<br>0 | 2<br>0<br>0 |
| 29 | 100<br>50<br>25 | 5<br>5<br>5 | 5<br>5<br>5 | 5<br>5<br>5 | 2<br>0<br>0 | 1<br>0<br>0 | 2<br>0<br>0 | 1<br>0<br>0 |
| 30 | 100<br>50<br>25 | 5<br>5<br>5 | 5<br>5<br>5 | 5<br>5<br>5 | 2<br>1<br>0 | 2<br>1<br>0 | 2<br>1<br>0 | 3<br>2<br>0 |
| 31 | 100<br>50<br>25 | 5<br>5<br>5 | 5<br>5<br>5 | 5<br>5<br>5 | 2<br>0<br>0 | 2<br>0<br>0 | 1<br>0<br>0 | 1<br>0<br>0 |
| 32 | 100<br>50<br>25 | 5<br>5<br>5 | 5<br>5<br>5 | 5<br>5<br>5 | 2<br>1<br>0 | 2<br>1<br>0 | 2<br>0<br>0 | 2<br>1<br>0 |

Test 3: Tests for phytotoxicity to crop plants (foliage treatment):

Each plastic box having a length of 15 cm, a width of 22 cm. and a depth of 6 cm was filled with a sterilized diluvium soil and seeds of cotton, soybean, radish, cabbage and eggplant were sown in a form of spots in a depth of about 1.5 cm. When the plants were grown to leaf-emergence stage, each solution of each herbicidal composition was uniformly sprayed to foliages at each dose of each active ingredient shown in Table 4. The solution was prepared by diluting, with water, a wettable powder, an emulsifiable concentrate or a solution described in examples of the composition except varying the active ingredient and the solution was uniformly sprayed by a small spray on all of foliages of the plants.

Two weeks after the spray treatment, the phytotoxicities to the plants were observed and rated by the following standard. The results are shown in Table 4.

Standard rating:

5: Complete death of plant
4: Serious phytotoxicity to plant
3: Fair phytotoxicity to plant
2: Slight phytotoxicity to plant
1: Only slight phytotoxicity to plant
0: Non phytotoxicity

Note:
Cot.: Cotton
Soy.: Soybean
Rad.: Radish
Cab.: Cabbage
Egg.: Eggplant

BAD ORIGINAL

Table 4

| Compound No. | Dose of Compound (g/a) | Cot. | Soy. | Rad. | Cab. | Egg. |
|---|---|---|---|---|---|---|
| 1 | 50<br>25 | 0<br>0 | 0<br>0 | 0<br>0 | 0<br>0 | 0<br>0 |
| 2 | 50<br>25 | 0<br>0 | 0<br>0 | 0<br>0 | 0<br>0 | 0<br>0 |
| 3 | 50<br>25 | 0<br>0 | 0<br>0 | 0<br>0 | 0<br>0 | 0<br>0 |
| 4 | 50<br>25 | 0<br>0 | 0<br>0 | 0<br>0 | 0<br>0 | 0<br>0 |
| 5 | 50<br>25 | 0<br>0 | 0<br>0 | 0<br>0 | 0<br>0 | 0<br>0 |
| 6 | 50<br>25 | 0<br>0 | 0<br>0 | 0<br>0 | 0<br>0 | 0<br>0 |
| 7 | 50<br>25 | 0<br>0 | 0<br>0 | 0<br>0 | 0<br>0 | 0<br>0 |
| 8 | 50<br>25 | 0<br>0 | 0<br>0 | 0<br>0 | 0<br>0 | 0<br>0 |
| 9 | 50<br>25 | 0<br>0 | 0<br>0 | 0<br>0 | 0<br>0 | 0<br>0 |
| 10 | 50<br>25 | 0<br>0 | 0<br>0 | 0<br>0 | 0<br>0 | 0<br>0 |
| 11 | 50<br>25 | 0<br>0 | 0<br>0 | 0<br>0 | 0<br>0 | 0<br>0 |
| 12 | 50<br>25 | 0<br>0 | 0<br>0 | 0<br>0 | 0<br>0 | 0<br>0 |
| 13 | 50<br>25 | 0<br>0 | 0<br>0 | 0<br>0 | 0<br>0 | 0<br>0 |
| 14 | 50<br>25 | 0<br>0 | 0<br>0 | 0<br>0 | 0<br>0 | 0<br>0 |
| 15 | 50<br>25 | 0<br>0 | 0<br>0 | 0<br>0 | 0<br>0 | 0<br>0 |

Table 4 (continued)

| Compound No. | Dose of Compound (g/a) | Cot. | Soy. | Rad. | Cab. | Egg. |
|---|---|---|---|---|---|---|
| 16 | 50<br>25 | 0<br>0 | 0<br>0 | 0<br>0 | 0<br>0 | 0<br>0 |
| 17 | 50<br>25 | 0<br>0 | 0<br>0 | 0<br>0 | 0<br>0 | 0<br>0 |
| 18 | 50<br>25 | 0<br>0 | 0<br>0 | 0<br>0 | 0<br>0 | 0<br>0 |
| 19 | 50<br>25 | 0<br>0 | 0<br>0 | 0<br>0 | 0<br>0 | 0<br>0 |
| 20 | 50<br>25 | 0<br>0 | 0<br>0 | 0<br>0 | 0<br>0 | 0<br>0 |
| 21 | 50<br>25 | 0<br>0 | 0<br>0 | 0<br>0 | 0<br>0 | 0<br>0 |
| 22 | 50<br>25 | 0<br>0 | 0<br>0 | 0<br>0 | 0<br>0 | 0<br>0 |
| 23 | 50<br>25 | 0<br>0 | 0<br>0 | 0<br>0 | 0<br>0 | 0<br>0 |
| 24 | 50<br>25 | 0<br>0 | 0<br>0 | 0<br>0 | 0<br>0 | 0<br>0 |
| 25 | 50<br>25 | 0<br>0 | 0<br>0 | 0<br>0 | 0<br>0 | 0<br>0 |
| 26 | 50<br>25 | 0<br>0 | 0<br>0 | 0<br>0 | 0<br>0 | 0<br>0 |
| 27 | 50<br>25 | 0<br>0 | 0<br>0 | 0<br>0 | 0<br>0 | 0<br>0 |
| 28 | 50<br>25 | 0<br>0 | 0<br>0 | 0<br>0 | 0<br>0 | 0<br>0 |
| 29 | 50<br>25 | 0<br>0 | 0<br>0 | 0<br>0 | 0<br>0 | 0<br>0 |
| 30 | 50<br>25 | 0<br>0 | 0<br>0 | 0<br>0 | 0<br>0 | 0<br>0 |

Table 4 (continued)

| Compound No. | Dose of Compound (g/a) | Cot. | Soy. | Rad. | Cab. | Egg. |
|---|---|---|---|---|---|---|
| 31 | 50<br>25 | 0<br>0 | 0<br>0 | 0<br>0 | 0<br>0 | 0<br>0 |
| 32 | 50<br>25 | 0<br>0 | 0<br>0 | 0<br>0 | 0<br>0 | 0<br>0 |

CLAIMS:

1. A quinoxaline derivative characterised by the general formula:

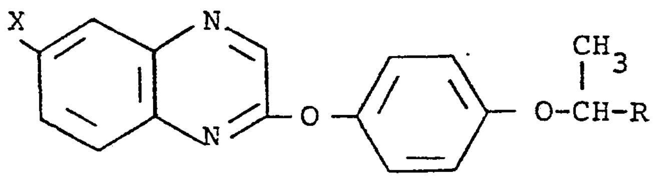

(I)

wherein X represents a $-CF_3$ or $-CH_3$ group and R represents a group of formula 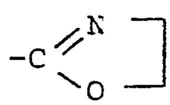 , 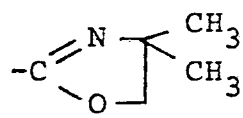 ,

$-CH=CHCOOR^2$, ($R^2$ represents a $C_1$ - $C_4$ alkyl group) -CN or $-CH_2OH$ or COR' wherein $R^1$ represents -OH, -O- alkali metal, -O-lower alkyl group, -O-halogenoalkyl group, -O-cyanoalkyl group, -O-lower alkoxyalkyl group, -O-lower alkenyl group, -O-lower alkynyl group, -O-cyclohexyl group, -O-phenyl group, 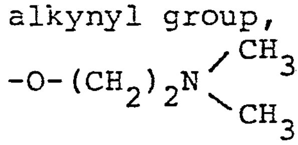 , 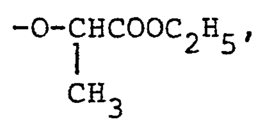,

$-O-(CH_2)_2SCH_3$, 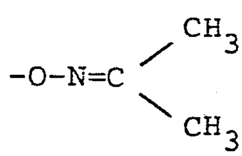 , $-S-R^3$ ($R^3$ represents a lower alkyl or phenyl group) or 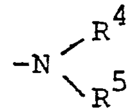 ($R^4$ and $R^5$ respectively represent a hydrogen atom, a lower alkyl group, a phenyl group or $-CH_2COOC_2H_5$).

2. A quinoxaline derivative according to claim 1, characterised by the formula

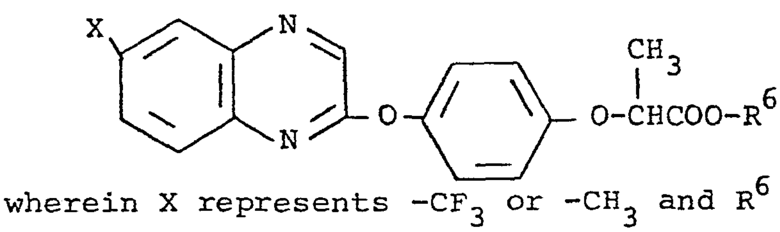

wherein X represents $-CF_3$ or $-CH_3$ and $R^6$ represents a hydrogen atom or a lower alkyl group, a lower alkoxyalkyl group, a lower alkenyl group, a lower alkynyl group, a phenyl group or a cyclohexyl group.

3. A quinoxaline derivative according to claim 1, characterised by the formula:

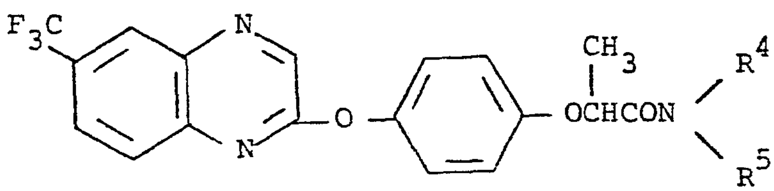

wherein $R^4$ and $R^5$ respectively represent a hydrogen atom, a lower alkyl group, a phenyl group or $-CH_2COOC_2H_5$.

4. A quinoxaline derivative according to claim 1, characterised by the formula:

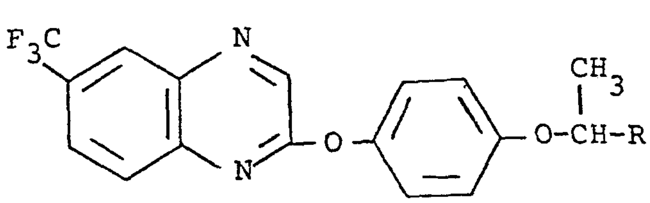

wherein R represents 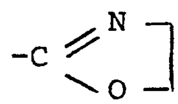 or 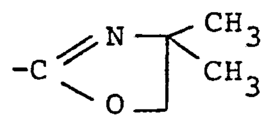

5. A quinoxaline derivative according to claim 1, characterised by the formula:

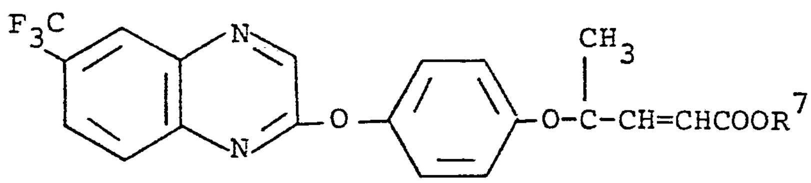

wherein $R^7$ represents a lower alkyl group.

6. A herbicidal composition which comprises a herbicidally active compound and an agricultural adjuvant, characterised in that the active ingredient is a quinoxaline derivative as defined in any preceding claim.

7. A herbicidal composition according to claim 6, characterised in that it comprises 0.5 to 95 wt.% of the active ingredient and 99.5 to 5 wt.% of the adjuvant.

8. A herbicidal composition according to claim 6 or claim 7, characterised in that it is in a form of a solution, an emulsifiable concentrate or a wettable powder.

0046468

European Patent Office

# EUROPEAN SEARCH REPORT

Application number

EP 80 30 2909

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| X | BE - A - 881 815 (NISSAN CHEMICAL INDUSTRIES LTD.)<br>* Pages 54,55; claims *<br>& GB - A - 2 042 539<br>-- | 1-8 | C 07 D 241/44<br>413/12<br>A 01 N 43/60<br>43/76 |
| | FR - A - 2 109 005 (UGINE KUHLMANN)<br>* Whole patent *<br>-- | 1 | |
| D | US - A - 4 130 413 (R. HANDTE)<br>* Whole patent *<br>& JP - A - 53 040 767/1978<br>-- | 1-8 | TECHNICAL FIELDS SEARCHED (Int. Cl.3)<br>C 07 D 241/44<br>413/12<br>A 01 N 43/60<br>43/76 |
| E | EP - A - 0 023 785 (ICI AUSTRALIA)<br>* Pages 9-11, 53-63 *<br>---- | 1-8 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 13-04-1981 | ALLARD |

EPO Form 1503.1 06.78